# EUROPEAN PATENT APPLICATION

(11) **EP 4 520 188 A1**
(43) Date of publication of application: **12.03.2025**
(21) Application number: 23799721.8
(22) Date of filing: 04.05.2023
(51) Int. Cl.: A23L 11/50, A23L 29/00, C12N 1/20, A23L 5/10, A23L 23/00, A23L 7/104

(54) **METHOD FOR PREPARATION OF TEMPEH, TEMPEH PREPARED THEREBY, SAUCE CONTAINING PREPARED TEMPEH, AND PREPARATION METHOD THEREFOR**

(30) Priority: 06.05.2022 KR 20220056156; 03.05.2023 KR 20230058023
(71) Applicant: CJ CheilJedang Corporation, Seoul 04560 (KR)
(72) Inventor: KIM, Hee Jin, Seoul 04560 (KR); CHO, Sun A, Seoul 04560 (KR); JEON, Jin, Seoul 04560 (KR); LEE, Eun Ju, Seoul 04560 (KR)
(74) Representative: Cabinet Laurent & Charras
(86) International application number: PCT/KR2023/006157
(87) International publication number: WO 2023/214840

(57) **Abstract**

Provided are a method of producing tempeh appropriate for mass production, tempeh produced thereby, and aged fermented tempeh.

## Description

### TECHNICAL FIELD

### [Cross-citation with related applications]

This application claims priority to Korean Patent Application No. 10-2022-0056156, filed on May 6, 2022, and Korean Patent Application No. 10-2023-0058023, filed on May 3, 2023, the disclosures of which are incorporated herein by reference.

### [TECHNICAL FIELD]

The present disclosure relates to a method of producing tempeh, tempeh produced by the method, a sauce including the tempeh, and a method of producing the sauce.

### BACKGROUND

Tempeh is a traditional Indonesian food made from fermented beans, and generally, the production method thereof includes boiling and peeling beans, inoculating Rhizopus strains mainly, and carrying out fermentation. Tempeh is made from beans like tofu, but its nutritional characteristics and texture are different from those of tofu. For example, its shape is similar to that of tofu, but its taste is similar to that of mushrooms. In the fermentation process for producing tempeh, as Rhizopus strains grow, the mycelium envelops the beans and clumps together to make a hard tissue, and thus, tempeh is also used as a meat substitute.

In Indonesia, tempeh is an everyday food ingredient which is cut and fried or sauteed. In addition, tempeh contains abundant amino acids and vitamins, and in particular, usually contains calcium in 2/3 of a cup of milk.

Currently, since tempeh is produced by a conventional method such as handwork rather than a mass production method and is produced in the form of being wrapped in banana leaf or plastic during the production, there is a possibility of cross-contamination in the production process and the production is not appropriate for mass production.

Therefore, the present inventors made diligent research efforts to develop a production method for mass production of tempeh which is nutritionally superior and a sauce using the tempeh produced in this manner, and as a result, completed the present disclosure.

### DISCLOSURE OF THE INVENTION

### TECHNICAL PROBLEM

An object of the present disclosure provides a method of producing tempeh for mass production.

Another object of the present disclosure provides aged tempeh having excellent flavor by aging tempeh.

Still another object of the present disclosure provides a sauce including aged tempeh.

### TECHNICAL SOLUTION

In order to achieve the above objects,

An aspect of the present disclosure provides a method of producing tempeh including: (a) peeling beans; (b) steaming the peeled beans; (c) inoculating a tempeh fungus into the steamed beans; and (d) fermenting the steamed beans inoculated with the tempeh fungus.

Another aspect of the present disclosure provides a method of producing aged tempeh includes aging the tempeh.

Still another aspect of the present disclosure provides a sauce includes the aged tempeh.

Hereinafter, the present disclosure will be described in detail.

### 1. Method of producing tempeh

An aspect of the present disclosure provides a method of producing tempeh including: (a) peeling beans; (b) steaming the peeled beans; (c) inoculating a tempeh fungus into the steamed beans; and (d) fermenting the steamed beans inoculated with the tempeh fungus.

The method of producing tempeh includes (a) peeling beans.

In the present disclosure, beans are fruits of an annual plant in the family fabaceae under the order rosales of the dicotyledons. Beans is characterized by containing abundant protein and lipids, and also vitamins A, B, D, and E. Beans used in producing the tempeh may be soybeans, white beans, black beans, chick peas, peas, black soybeans, kidney beans, green beans, green pod kidney beans, yellow kidney beans, red flower kidney beans, lentils, pintos, and the like. Specifically, the beans may be round-shaped round soybeans, sections or pressing pieces of round soybeans, or ground round soybeans.

By peeling the beans, the hyphae of a tempeh fungus penetrates deep into the cotyledons of beans and facilitates growth. The peeling step may be performed using a peeler, for example, a rubber roller mill, a burr mill, a corn mill, a steel roller mill, and the like. When beans are peeled, moisture easily penetrates the inside of the beans to shorten a steaming time, thereby improving process efficiency. In a conventional method of producing tempeh, peeling is performed in a manner of steaming raw material beans and then rubbing or stepping the steamed beans, but unlike this, when bean skins are removed before steaming the beans as in the present disclosure, sterilization is performed by steaming after peeling, and thus, a possibility of cross-contamination is much reduced and production efficiency is improved.

The step of peeling beans may be performed by dry peeling. In addition, the dry peeling may be performed at room temperature without heating. When the raw material beans are peeled in a dry peeling manner, the raw material beans may be peeled without a heating process or a process of soaking in water, and thus, the process is simplified and a time for subsequent soaking step or steaming step is shortened, thereby increasing the efficiency of a production process.

The production method may further include soaking the peeled beans before steaming.

The step of soaking beans is a process of hydrating beans, and water is added before steaming to soak beans so that a moisture content is appropriate for tempeh production. A time to perform soaking may be in a range selected from any one lower limit selected from the group consisting of 10 minutes, 30 minutes, 1 hour, 2 hours, 3 hours, 4 hours, and 5 hours and any one upper limit selected from the group consisting of 6 hours, 8 hours, 10 hours, 15 hours, 20 hours, 24 hours, and 48 hours, and specifically, the step may be performed for 2 hours to 24 hours.

Compared with the case of soaking unpeeled beans, the soaking time to reach a target moisture content may be shortened by soaking peeled beans.

The method of producing tempeh includes (b) steaming the peeled beans.

The step of steaming the peeled beans refers to a process of applying heat to beans by steam to cook the beans. The step of steaming the beans may kill microorganisms of beans, destruct a trypsin inhibitor or other nutrient-inhibiting materials, and increase nutrients required to grow a tempeh fungus. In general, a process of steaming beans may prevent loss of water soluble nutrients of beans as compared with boiling in boiling water.

In an exemplary embodiment, the steaming step may be performed by a steam pressurization method. The steam pressurization method may use saturated steam in which water and steam are in equilibrium, and since the saturated steam emits heat without taking away moisture of beans, beans may be rapidly steamed using saturated steam. When steaming is performed by the pressurization method of saturated steam, the pressure may be 0.5 kgf/cm² to 3 kgf/cm², 0.6 kgf/cm² to 2.7 kgf/cm², 0.7 kgf/cm² to 2.5 kgf/cm², 0.8 kgf/cm² to 2.3 kgf/cm², or 1.0 to 2.0 kgf/cm². In addition, the saturated steam may be at a temperature of 60°C to 120°C, 70°C to 110°C, or 80°C to 100°C. Herein, the steaming by a steam pressurization method may use a pressurization-type steamer, for example, a rotary pressurization-type steamer (NK-type steamer) or batch pressurization steamer.

In an example, when soybeans are steamed by a steam pressurization method, it was confirmed that tempeh fungus growth is smooth and a food poisoning bacteria or common microorganism level is decreased, as compared with the case of producing soybean tempeh by steaming soybeans by a conventional boiling method.

In the steam pressurization method, a steaming time in producing beans in the state of being appropriate for tempeh production may be shortened to 20 minutes or less, 17 minutes or less, 15 minutes or less, 13 minutes or less, 10 minutes or less, 8 minutes or less, 6 minutes or less, 5 minutes or less, or 3 minutes or less. Since the steam pressurization method has an effect of shortening the time to steam beans, it improves process efficiency, as compared with a boiling method or a simple steaming method. In the steaming step, the pressure, the temperature, and the steaming time of the saturated steam pressurization method may vary depending on the moisture content of beans and the like.

The moisture content of the steamed beans may be 30 wt% to 80 wt%, 40 wt% to 75 wt%, 42 wt% to 73 wt%, 45 wt% to 73 wt%, 47 wt% to 70 wt%, specifically 50 wt% to 65 wt%. When the steamed beans have the moisture content in the above range, the smooth growth of a tempeh fungus may be promoted to produce tempeh having excellent quality.

The method of producing tempeh includes (c) inoculating a tempeh fungus into the steamed beans.

The tempeh fungus is the strain of the genus *Rhizopus,* and for example, may be *Rhizopus oligosporus.* The tempeh fungus is an edible mushroom fungus which grows naturally on banana leaves, contains a large amount of superdismutase which decomposes lipids which are not good for the human body, and has an effect of activating intestinal immunity to inhibit malignant Escherichia coli and the like. The inoculation of the tempeh fungus may be addition of a tempeh starter including the tempeh fungus. The tempeh starter may be obtained by artificially inoculating purely cultured Rhizopus strains into steamed grains and culturing the grains, and for example, may be in the form of a mixture of flour or rice flour and a tempeh fungus or in the form of a seed koji in which a Rhizopus fungus is sporulated in flour or rice flour, and for example, may be a mixture of 90-99.9 wt% of rice flour and 0.1-10 wt% of a tempeh fungus, 95-99.9 wt% of rice flour and 0.1-5 wt% of a tempeh fungus, or 90-99.9 wt% of rice flour and 0.1-10 wt% of a tempeh fungus. In step (c), the content of a tempeh fungus starter which is added to the steamed beans may be 1 wt% to 10 wt%, 1 wt% to 9 wt%, 1 wt% to 8 wt%, 2 wt% to 8 wt%, 3 wt% to 7 wt%, or 4 wt% to 6 wt% with respect to the weight of the steamed beans. The content of a tempeh fungus inoculated into the steamed beans may be 0.01 wt% to 0.1 wt%, 0.01 wt% to 0.09 wt%, 0.01 wt% to 0.08 wt%, 0.02 wt% to 0.08 wt%, 0.03 wt% to 0.07 wt%, or 0.04 wt% to 0.06 wt% with respect to the weight of the steamed beans.

As the inoculation amount of the tempeh fungus increases, the tempeh fungus multiplies predominantly, and thus, the growth of pathogenic microorganisms including common microorganisms or food poisoning bacteria is inhibited and tempeh having excellent quality may be produced.

The method of producing tempeh includes (d) fermenting the steamed beans inoculated with the tempeh fungus.

The step of fermenting the steamed beans inoculated with the tempeh fungus may refer to a step of culturing beans inoculated with the tempeh fungus so that the tempeh fungus may multiply. The tempeh fungus culture may be performed at an appropriate temperature and humidity. For example, the fermentation may be performed in a temperature range selected from any one lower limit selected from the group consisting of 10°C, 15°C, 18°C, 20°C, 22°C, 24°C, 25°C, 26°C, 28°C, and 30°C and any one upper limit selected from the group consisting of 30°C, 32°C, 33°C, 35°C, 38°C, 40°C, 43°C, 45°C, and 50°C. For example, it may be performed at 15°C to 45°C, 18°C to 43°C, 20°C to 40°C, 22°C to 38°C, or 25°C to 35°C.

In addition, a fermentation time may be 10 hours to 15 days, 15 hours to 15 days, 20 hours to 15 days, 1 day to 15 days, 1 day to 14 days, 1 day to 13 days, 1 day to 12 days, 1 day to 11 days, 1 day to 10 days, 1 day to 9 days, 1 day to 8 days, 1 day to 7 days, 1 day to 6 days, 1 day to 5 days, 1 day to 4 days, 1 day to 2 days, or 2 days to 4 days. In addition, a humidity during the fermentation may be 40% to 90%, 40% to 85%, 40% to 80%, 40% to 70%, 50% to 70%, 50% to 80%, 60% to 80%, 55% to 65%, or 65% to 75%. When the fermentation is performed in the above conditions, the growth of a tempeh fungus predominates and microbial growth is inhibited, thereby improving the storage stability of tempeh excellently. In addition, the tempeh produced in the fermentation conditions has uniformly improved maturation and good quality, and thus, is suitable for mass production.

The production method may further include cooling before fermenting the steamed beans. The cooling step may be performed in a product temperature range selected from any one lower limit selected from the group consisting of 10°C, 15°C, 18°C, 20°C, 22°C, 24°C, 26°C, 28°C, and 30°C and any one upper limit selected from the group consisting of 30°C, 32°C, 33°C, 35°C, 38°C, 40°C, 45°C, and 50°C so that the temperature is appropriate for fermentation. By cooling the steamed beans as described above, the oversteaming of beans by residual heat is prevented and an environment in which the tempeh fungus grows well may be created.

The steamed beans may be stirred or crushed into a smaller size for the convenience of tempeh fungus inoculation and tempeh molding before inoculating a tempeh fungus.

The production method may further include adding an acid to the steamed beans in the step of (c) inoculating a tempeh fungus into the steamed beans.

The acid refers to a material which is acidic and tastes sour when it is dissolved in water, and a usable acid may be, for example, vinegar, citric acid, lactic acid, lemon juice, malic acid, gluconic acid, and the like, but is not limited thereto, and is not limited as long as it is an edible acid. By adding the acid, acidity appropriate for culturing a tempeh fungus may be maintained and the tempeh fungus grows predominantly, so that the growth of other common microorganisms, fungi, and pathogenic microorganisms is inhibited. The acidity may be acidity at which the growth of a tempeh fungus is not inhibited and the growth of other microorganisms, for example, pathogenic microorganisms or fungi is not inhibited. The acidity (%) of the acid refers to the content (g) of the acid included in 100 mL of an acid solution, and the acidity may be in a range selected from any one lower limit selected from the group consisting of 5%, 6%, 7%, 9%, and 10%; and any one upper limit selected from the group consisting of 12%, 13%, 15%, 16%, 17%, 18%, 19%, 20%, and 25%, and specifically, the acidity may be in a range of 5% to 20%, 7% to 19%, 9% to 15%, 10% to 13%, or 10% to 12%. The acid may be added when the product temperature of the steamed beans is 50°C or lower.

In the step of adding an acid to the steamed beans in step (c), the acid may be inoculated into the steamed beans at a rate of 1 wt% to 10 wt%, 1 wt% to 9 wt%, 1 wt% to 8 wt%, 2 wt% to 8 wt%, 3 wt% to 7 wt%, or 4 wt% to 6 wt% with respect to the weight of raw material beans. However, the amount is not limited thereto and may correspond to any amount to create the environment to grow a tempeh fungus.

In an exemplary embodiment, when the acid is added at a rate of 4 wt% to 6 wt% with respect to the total weight of the raw material beans, it was confirmed that microbial growth was more inhibited as compared with the case of adding the acid at 2 wt%. In addition, when the acidity is identically maintained regardless of the type of added acid, it was confirmed that microbial growth was inhibited to the same level. The microorganisms of which the growth is inhibited may be microorganisms other than harmful microorganisms or a tempeh fungus.

In an exemplary embodiment, the step of adding an acid to the steamed beans in step (c) may be performed before, after, or at the step of inoculating a tempeh fungus into the steamed beans.

The production method may further include molding the steamed beans inoculated with the tempeh fungus in the step of (c) inoculating a tempeh fungus into the steamed beans. In the specification of the present application, the steamed beans inoculated with the tempeh fungus may be referred to as "tempeh" even before fermenting the beans. The tempeh (steamed beans inoculated with the tempeh fungus) may be molded so that a height is 2 cm to 5 cm or 3 cm to 4.5 cm. When the height of tempeh is in the above range, the growth of a tempeh fungus is facilitated so that the mycelium extends evenly to a deep part so that fermentation is well done, and thus, tempeh having excellent quality which is hard, has good texture, and has a low microbial level may be efficiently produced.

In the step of (d) fermenting the steamed beans inoculated with the tempeh fungus, the beans inoculated with the tempeh fungus may be fermented after putting the beans in a perforated container made of any one or more materials selected from the group consisting of polyethylene (PE), polyethylene vinyl, or silicon. When the container made of the material is used, heating may be easily adjusted and temper having good quality which is fermented well by a tempeh fungus may be produced. In addition, the container may be perforated. Since air may circulate by perforation in the container, the container has high oxygen permeability and may perform fermentation by a tempeh fungus effectively.

In an example, when a tray made of polyethylene was used, it was confirmed that heating may be easily adjusted during fermentation and soybean tempeh and wheat tempeh having good quality which were well fermented by a tempeh fungus may be produced.

In another aspect of the present disclosure, tempeh produced by the method of producing tempeh is provided.

The tempeh produced by the production method may have a moisture content of 20% to 45%, 25% to 40%, 26% to 39%, 27% to 38%, 28% to 39%, 29% to 38%, or 30% to 36% with respect to the total weight of tempeh. When the moisture content included in tempeh is in the range described above, microbial stability is excellent.

In the tempeh produced by the production method, growth of microorganisms is inhibited by dominant growth of a tempeh fungus, and thus, the tempeh has better storage stability and distribution stability than conventional handmade tempeh.

The microorganisms of which the growth is inhibited may be *Bacillus cereus,* common bacteria, fungi, and the like. The *Bacillus cereus* is a kind of soil bacterium and is widely distributed in nature such as dust, sewage, and streams including a human living environment, and is called food poisoning bacteria, as bacteria which cause decay of plants and animals and disease in human and animals. In an example, the growth of a tempeh fungus predominates in the tempeh produced by the production method, and *Bacillus cereus* bacteria may be detected at 10⁸ CFU/g or less, 10⁷ CFU/g or less, 10⁶ CFU/g or less, or 10⁵ CFU/g or less. The common bacteria may be detected at 10¹⁰ CFU/g or less, 10⁹ CFU/g or less, or 10⁸ CFU/g or less.

In an example, when a tempeh fungus starter is added at 4 wt% to 6 wt% with respect to the weight of the raw material beans, the growth of a tempeh fungus predominates, and thus, the *Bacillus cereus* bacteria may be detected at 10⁵ CFU/g or less and common bacteria may be detected at 10¹⁰ CFU/g or less.

In an example, when a tempeh fermentation temperature is 25°C to 35°C, common bacteria may be detected at 10¹⁰ CFU/g or less.

In an example, when a tempeh fermentation humidity is 65% to 75%, the *Bacillus cereus* bacteria may be detected at 10⁵ CFU/g or less and the common bacteria may be detected at 10⁹ CFU/g or less.

The tempeh produced as described above is fermented food, and generally, it is eaten by heating. The tempeh produced by the production method of the present disclosure may be used as an aged product which has been further aged as described later or a sauce including an aged product. When tempeh is produced as aged tempeh or a sauce including the same, a composition for controlling microorganisms such as an alcohol, a natural antibacterial agent, and a preservative is added or an additional sterilization process such as a heating process is performed, thereby maintaining the microbial stability.

### 2. Method of producing aged tempeh

Another aspect of the present disclosure provides a method of producing aged tempeh including: (a) producing tempeh by the method of producing tempeh described above; and
(b) an aging step of aging a mixture in which at least one or more of saline and brine are added to the produced tempeh.

In step (a) of the method of producing aged tempeh, a step of producing tempeh is producing tempeh by the method of producing bean tempeh described above, and as described above, may include peeling beans; steaming the peeled beans; inoculating a tempeh fungus into the steamed beans; and fermenting the steamed beans inoculated with the tempeh fungus. Since the details of the method of producing tempeh are the same as those in the method of producing tempeh described above, it is cited and the description is not duplicated.

The method of producing aged tempeh includes (b) adding one or more of saline and brine to tempeh and aging the mixture.

The aging is fermenting a mixture to which saline or brine is added according to a fermented flavor to be desired to produce the aged tempeh. The aged tempeh is produced by including a step of aging tempeh which is a fermented food, and thus, has an increased unique flavor of tempeh as compared with the tempeh itself and also has an effect of much increasing contents of amino acid, vitamins, and the like. The effect of the aged tempeh is not limited, but for example, tempeh produced by first fermentation by *Rhizopus oligosporus* strains is subjected to second fermentation, thereby further increasing the content of useful components of soybeans, such as amino acids, free sugars, and organic acids to further increase its nutritional value.

The aging step may include stirring the mixture. The stirring may be performed by a stirrer, for example, an impeller, and a stirring speed may be 10 to 500 rpm, 25 to 450 rpm, 30 to 400 rpm, or specifically 50 to 300 rpm. The stirring may be performed for 1 minute to 1 hours once a day, twice a day, or continuously during an aging period.

By performing aging by stirring, decomposition of a protein component in the mixture is promoted to increase the content of amino acid and the like to improve a flavor and increase an aging degree.

The moisture content of the mixture may be 40 to 80 wt%, 45 to 75 wt%, 50 to 70 wt%, 55 to 65 wt%, or 50 to 60 wt%. In addition, in the aging step, one or more of the saline or brine may be added so that a salt content is 5 to 15 wt%, for example, 5 to 10 wt%, 6 to 9 wt%, or 7 to 9 wt% with respect to the total weight of the mixture. When the content of moisture and/or salt in the mixture is within the range, the nutritional component of the produced aged tempeh, specifically, an amino acid content may be improved excellently, and the microbial growth in the produced aged tempeh is inhibited to improve storage stability of food excellently.

The aging step may include further adding one or more of steamed beans, steamed wheat rice, and steamed rice to the mixture. In a specific example, the aging degree of the steamed soybeans, the steamed wheat rice, or the steamed rice may be added as extra rice to the aged tempeh to adjust an aging degree. One or more of the steamed beans, the steamed wheat rice, and the steamed rice may be added in a content range selected from any one lower limit selected from the group consisting of 1 wt%, 2 wt%, 3 wt%, 4 wt%, 5 wt%, 6 wt%, 7 wt%, 8 wt%, 10 wt%, 12 wt%, 14 wt%, 16 wt%, 18 wt%, and 20 wt%; and any one upper limit selected from the group consisting of 25 wt%, 30 wt%, 35 wt%, 40 wt%, 50 wt%, 60 wt%, 70 wt%, 80 wt%, and 90 wt%, with respect to the total weight of the mixture, but is not limited thereto and may be appropriately adjusted depending on the aging degree or flavor to be desired. The aging may be performed at an appropriate temperature for an appropriate period of time. For example, it may be performed at 10°C to 60°C, for example, performed at 15°C to 55°C, 20°C to 50°C, 25°C to 55°C, or 25°C to 35°C. In addition, the aging may include fermentation for 1 day to 15 days, 1 day to 14 days, 1 day to 13 days, 1 day to 12 days, 1 day to 11 days, 1 day to 10 days, 1 day to 9 days, 1 day to 8 days, 1 day to 7 days, 1 day to 6 days, 1 day to 5 days, 1 day to 4 days, or 2 days to 4 days, depending on temperature conditions. The aging may be performed at a low temperature for a long time or a high temperature for a short time, depending on the state of an aged product or a stirring rotation speed, whereby an aging degree of the aged product may be adjusted.

In addition, the aging step may be repeated once or more, twice or more, or three times or more, thereby adjusting the aging degree of the aged tempeh.

Another aspect of the present disclosure provides aged tempeh produced by the production method.

The content of amino-type nitrogen included in the aged tempeh may be 5 mg% or more and 1200 mg% or less, and specifically, in a range selected from a lower limit of 40 mg%, 80 mg%, 100 mg%, 150 mg%, 200 mg%, 250 mg%, 300 mg%, or 400 mg% and an upper limit of 1200 mg%, 1100 mg% or less, or 1000 mg%. More specifically, it may be 40 mg% to 1200 mg%, 100 mg% to 1100 mg%, or 200 mg% to 1000 mg%.

The amino-type nitrogen is an indicator of an aging degree which shows the content of amino acid produced by decomposition of protein by an enzyme produced by microorganisms by aging, and since its content is important for the taste of soy sauces such as umami, it is generally a management standard of soy sauce. The aged tempeh of the present disclosure may be produced into an aged product having a desired aging degree (content of an amino-type nitrogen) by adjusting an aging period, an aging temperature, and a rotation speed during stirring, depending on the type of sauces to be produced. For example, when red pepper pastes are to be produced with the aged tempeh, aged tempeh produced to have an aging degree of 100 to 300 mg% may be used, when ssamjang is to be produced, aged tempeh produced to have an aging degree of 220 mg% or more may be used, and when soybean sauce is to be produced, aged tempeh produced to have an aging degree of 400 mg% or more may be used. The aging degree may be appropriately adjusted by the type of raw material beans, an aging period, an aging temperature, an aging method, or an inoculation amount.

When the method of producing aged tempeh according to the present disclosure is used, the raw material of a new sauce which is rich in nutrients such as amino acids, free sugars, and organic acids and has enhanced flavor as compared with produced tempeh as well as raw material beans may be provided.

### 3. Sauce including tempeh or aged tempeh

In addition, another aspect of the present disclosure provides a sauce including the tempeh produced by the production method or the aged product thereof.

The sauce including the aged tempeh of the present disclosure may be used as a raw material for producing a new sauce beyond a simple food material, and thus, the field of application is broadened to increase tempeh utility.

The sauce including tempeh may include a content of 1 to 99 wt%, for example, 1 to 90 wt%, 1 to 80 wt%, 1 to 70 wt%, 1 to 60 wt%, 1 to 50 wt%, 1 to 40 wt%, 1 to 35 wt%, 1 to 30 wt%, 1 to 10 wt%, or 3 to 27 wt% of the tempeh.

The sauce including tempeh may be the tempeh or aged tempeh simply diluted in water, but for enhancing the flavor of the sauce including tempeh, one or more additive selected from the group consisting of spice plants, processed spices, pastes, sugars, flavor enhancers, salt, vinegar, nuts, composite seasoned foods, and fermented bean foods are mixed with the aged tempeh, and thus, it may further include one or more selected from the group consisting of spice plants, processed spices, pastes, sugars, flavor enhancers, salt, vinegar, nuts, composite seasoned foods, and fermented bean foods.

The "spice plant" is a plant to add for adding the taste and flavor of a sauce, and though it is not limited thereto, it may include chili pepper, garlic, ginger, black pepper, onion, green onion, shallot, and the like.

The "processed spice" is obtained by simply processing leaves, stems, fruits, roots, or the like of spice plants or mix-processing food additives, and is used for increasing other food flavors. The processed spice is not limited, but may include, for example, natural spices such as red pepper powder, black pepper powder, cinnamon powder, oregano hall, and rosemary, and spice preparations such as mustard, curry, and tomato ketchup.

The "composite seasoned food" is processed into powder, granules, or a solid shape by mixing food with sugars, salt, spices, protein hydrolysate, yeast or its extracts, or food additives, and for example, a chili pepper seasoning may be used, and the chili pepper seasoning is a paste made by mixing chili pepper powder, onion, garlic, water, salt, and the like.

The "bean fermented food" is a product obtained by mixing and fermenting beans, specifically soybeans or soybean cake and sugar, and is a food having liquid physical properties having a sugar content of 30% or more.

The "pastes" are obtained by manufacturing/processing aspergillus culture in animal/vegetable raw materials or fermented/aged mixture of fermented soybean lump as a raw material, salt, and the like, and though they are not limited, for example, they include Korean fermented soybean lump, modified soybean lump, Korean soy sauce, brewed soy sauce, acid-decomposed soy sauce, enzyme-decomposed soy sauce, mixed soy sauce, Korean soybean paste, soybean paste, chili pepper paste, chunjang, fast-fermented bean paste, mixed soybean paste, and the like.

The "sugars" represent sugars obtained by processing starch raw materials or sugar solution, sugar syrups, oligosaccharides, glucose, fructose, taffy, or sugar products processed therefrom.

The "flavor enhancer" is for enhancing the taste or flavor of food, and may include, for example, sodium glutamate and the like.

The "nuts" are not limited as long as they are edible nuts, and may include, for example, peanuts, almonds, walnuts, macadamia, hazelnuts, pine nuts, acorns, nutmegs, Brazil nuts, cashews, coffee beans, cocoa beans, pistachios, pecans, sunflower seeds, and the like.

In a specific example, the tempeh-containing sauce may further include at least one or more of chili pepper powder, chili pepper, salt, soy sauce, tomato ketchup, and other spice plants, processed spices, sugars, flavor enhancers, composite seasonings, and nuts.

The sauce including tempeh may not include additives other than the components described above, or may further include other additives. When other additives are further included, such additives may include preservatives and/or excipient acceptable for food.

The preservatives acceptable for food are not limited, and may include, for example, sorbic acids, benzoic acids, dehydroacetic acids, paraoxybenzoic acids, propionic acids, and the like. The preserves may use, for example, a salt form such as potassium sorbate, calcium sorbate, sodium benzoate, sodium propionate, calcium propionate, and sodium paraoxybenzoate.

The excipients acceptable for food is not limited, and may be, for example, at least one selected from the group consisting of crosslinked sodium carboxymethylcellulose, gum ghatti, persimmon colorant, licorice extract, formic acid, geranyl formate, citronellyl formate, isoamyl formate, gum resin, geraniol, crystalline cellulose, cinnamic acid, methyl cinnamate, ethyl cinnamate, cinnamic aldehyde, cinnamon alcohol, kaoliang colorant, benzoyl peroxide, hydrogen peroxide, acetic acid peroxide, ammonium persulfate, guar gum, disodium 5'-guanylate, citric acid, manganese citrate, trisodium citrate, sodium ferrous citrate, iron citrate, ammonium iron citrate, potassium citrate, calcium citrate, magnesium silicate, calcium silicate, silicon resin, diatomaceous earth, gluconic acid, sodium gluconate, copper gluconate, magnesium gluconate, manganese gluconate, zinc gluconate, iron gluconate, potassium gluconate, calcium gluconate, glutaminase, butyric acid, butyl butyrate, ethyl butyrate, isoamyl butyrate, neotame, nisin, nicotinic acid, nickel, nicotinamide, dextranase, dextran, sodium lauryl sulfate, lactase, lactoferrin concentrate, lactitol, lecithin, rosin, locust bean gum, rutin, linalool, mannitol, maltol, D-maltitol, sodium metasilicate, sodium metaphosphate, potassium metadiate, sodium metabisulfite, potassium metabisulfite, sodium methoxide, anhydrous sulfurous acid, myristic acid, microfibrillar cellulose, vanillin, white clay, betaine, bentonite, powdered cellulose, sodium fluoride, biotin, vitamins, glacial acetic acid, DL-malic acid, sodium saccharin, saffron colorant, acid clay, acidic sodium sulfite, acidic sodium aluminum phosphate, acidic sodium pyrophosphate, acidic calcium pyrophosphate, magnesium oxide, zinc oxide, calcium oxide, methyl salicylate, ferric oxide, fiber wax, sodium hydroxide, potassium hydroxide, calcium hydroxide, magnesium hydroxide, sucralose, shellac, steviol glycosides, stearic acid, stearates, food coloring, benzoic acid, benzoates, alginic acid and alginate, inositol, silicon dioxide, chlorine dioxide, carbon dioxide, titanium dioxide, xanthan gum, starch, modified starch, lactic acid and lactates, gelatin, gellan gum, seed koji, carnauba wax, carrageenan, gum karaya, carotene, carboxymethylcellulose sodium, carboxymethylcellulose calcium, carboxymethyl starch sodium, casein and caseinate salts, chitosan, chitin, tara gum, tamarind gum, taurine, tannic acid, palmitic acid, phenyl ethyl acetate, phenyl isobutyl acetate, pectin, pepsin, hydroxypropyl methyl cellulose, hydroxypropyl cellulose, hyaluronic acid, and yeast extract.

### ADVANTAGEOUS EFFECTS

Tempeh having an excellent nutritional value may be mass-produced by the method of producing tempeh of the present disclosure. The method of producing tempeh of the present disclosure is less likely to cross-contamination as compared with a conventional production method and may maintain tempeh quality uniformly. The method of producing tempeh of the present disclosure considers the characteristics of tempeh strains, and it has a low microbial level and excellent distribution stability and may efficiently mass produce hard tempeh having good quality.

In addition, compared with conventional tempeh produced only by soybeans, the method of producing tempeh of the present disclosure may use various types of beans. The present disclosure produces a sauce including aged tempeh, thereby using tempeh as a raw material for producing a novel sauce beyond conventionally using tempeh only as a food material to further broaden the field of application of tempeh and increase tempeh utility.

However, the effect of the present disclosure is not limited to the effects mentioned above, and other effects which are not mentioned herein may be clearly understood by a person skilled in the art from the following description.

### BRIEF DESCRIPTION OF THE DRAWINGS

FIG. 1 shows a method of producing soybean tempeh according to an exemplary embodiment of the present disclosure.
FIG. 2 shows a difference in microbial levels of soybean tempeh depending on added amounts of a tempeh starter.
FIG. 3 shows a difference in tempeh quality depending on heights of molded tempeh during tempeh molding.
FIG. 4 shows a difference in microbial levels of soybean tempeh depending on the fermentation condition (humidity) of tempeh.
FIG. 5 shows the quality of tempeh produced by performing tempeh fermentation in a relative humidity of 30%.
FIG. 6 shows a difference in microbial levels of soybean tempeh depending on the fermentation condition (temperature) of tempeh.

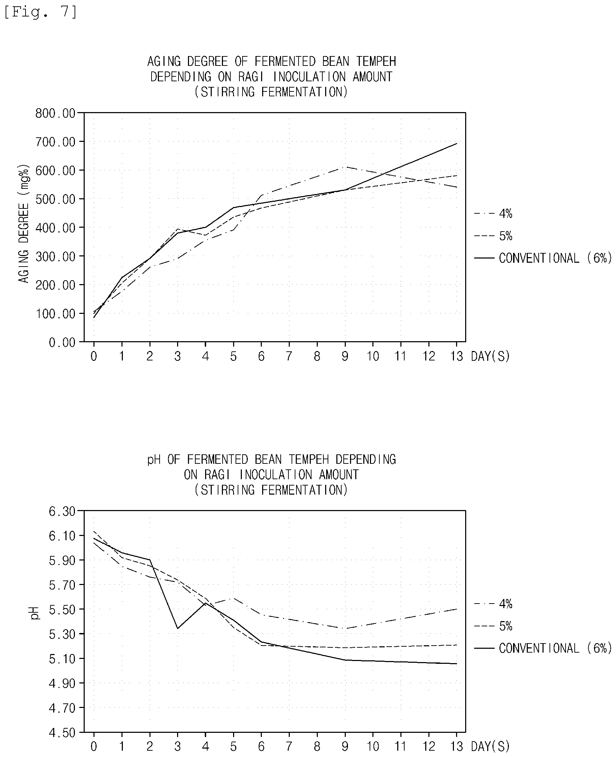

FIG. 7 shows results of comparison of the aging degrees and pHs of aged products obtained by aging soybean tempeh (bean tempeh) produced with different inoculation amounts of a tempeh fungus.
FIG. 8 shows results of comparison of the aging degrees and pHs of aged products obtained by aging soybean tempeh (bean tempeh) produced with different fermentation condition (relative humidity).
FIG. 9 shows results of comparison of the aging degrees and pHs of aged products obtained by aging soybean tempeh (bean tempeh) produced with different fermentation condition (temperature).
FIG. 10 shows results of comparison of the aging degree and pHs of tempeh aged depending on the type of raw material beans.

### MODE FOR CARRYING OUT THE INVENTION

Hereinafter, the present disclosure will be described in detail by the examples.

However, the following examples only specifically illustrate the present disclosure, and the content of the present disclosure is not limited by the examples.

### [Example 1] Production of soybean tempeh

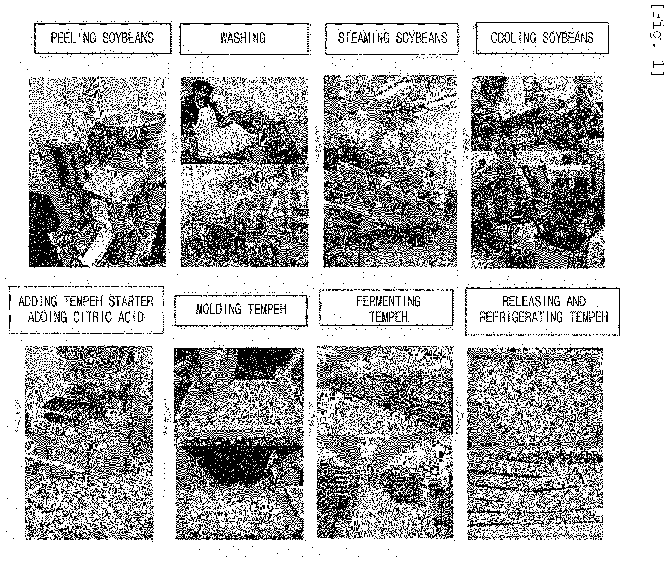

As shown in FIG. 1, raw grain soybeans which were neither heated nor hydrated were dry peeled using a peeler, and dust, bacteria, and foreign matter were removed from the soybeans by an aeration washing process. When the peeling was performed before steaming the raw grain soybeans, the peeling process of soybeans may be omitted after steaming soybeans, and thus, a possibility of cross-contamination may be reduced and process efficiency may be improved.

The washed soybeans were soaked in water for 2 hours, transferred to a rotatable NK steaming tank, and steamed by applying 1.0 to 2.0 kgf/cm² of saturated steam at about 90°C for 2 minutes and 30 seconds. The steamed soybeans were discharged from the steaming tank, and passed through a cooler to be cooled to a product temperature of 25 to 30°C.

When the soybeans were steamed by a steam pressurization method as described above, a steaming time was shortened as compared with the case of steaming by a boiling method, thereby improving process efficiency. In addition, since a cooling process was performed after the steaming, an oversteaming phenomenon by residual heat may be prevented.

Next, a tempeh starter including 99 wt% of rice flour and 1 wt% of *Rhizopus* was inoculated at 6 wt% with respect to the raw material soybeans into the steamed and cooled soybeans, 4 wt% of citric acid with respect to the weight of the raw material soybeans was added, and stirring was performed for 3 minutes to produce a mixture. The mixture was put into a perforated polyethylene tray and molded to have a height of 3 cm. The tray containing the molded mixture was covered with a perforated board, and the mixture was fermented for 24 hours to 29 hours in a fermentation room in which air was circulated and a temperature of 27°C and a humidity of 60% were maintained, thereby producing soybean tempeh. The produced tempeh was kept refrigerated at 5°C for 7 days.

### [Example 2] Production of aged soybean tempeh

Brine was added to the soybean tempeh produced in Example 1 so that a moisture content was adjusted to about 60%, and aging was performed while performing stirring at a speed of 50 to 300 rpm, thereby producing aged soybean tempeh. Soybean tempeh produced by changing an added amount of tempeh starter, fermentation humidity, and fermentation temperature in the soybean tempeh production was further aged to produce an aged product, and the amino-type nitrogen content and pH of the produced aged product were measured, thereby evaluating the effect of the production conditions during the soybean tempeh production on the aging degree of the aged product. The results are shown in the following Experimental Example 2.

### [Example 3] Production of black bean tempeh

Raw grain black beans which were neither heated nor hydrated were dry peeled using a peeler, and dust, bacteria, and foreign matter were removed from the black beans by an aeration washing process.

The washed black beans were soaked in water for 2 hours, transferred to a rotatable NK steaming tank, and steamed by applying 1.0 to 2.0 kgf/cm² of saturated steam at about 90°C for 2 minutes and 30 seconds. The steamed black beans were discharged from the steaming tank, and passed through a cooler to be cooled to a product temperature of 25 to 30°C.

Next, a tempeh starter including 99 wt% of rice flour and 1 wt% of *Rhizopus* was inoculated at 6 wt% with respect to the raw material black beans into the steamed and cooled black beans, 4 wt% of citric acid with respect to the weight of the raw material black beans was added, and stirring was performed for 3 minutes to produce a mixture. The mixture was put into a perforated polyethylene tray and molded to have a height of 3 cm. The tray containing the molded mixture was covered with a perforated board, and the mixture was fermented for 24 hours to 29 hours in a fermentation room in which air was circulated and a temperature of 27°C and a humidity of 60% were maintained, thereby producing black bean tempeh. The produced black bean tempeh was kept refrigerated at 5°C for 7 days.

### [Experimental Example 1] Evaluation of quality depending on method of producing soybean tempeh

### 1-1. Evaluation of quality of soybean tempeh depending on inoculation amount of tempeh fungus

Soybean tempeh was produced in the same manner as in Example 1, except that the added amount of tempeh fungus starter was changed to 3 wt% to 6 wt% with respect to the total weight of the raw grain soybeans. *Bacillus cereus* which is a food poisoning bacteria of the produced soybean tempeh and the total bacterial count of common bacteria were measured and microbial levels were compared.

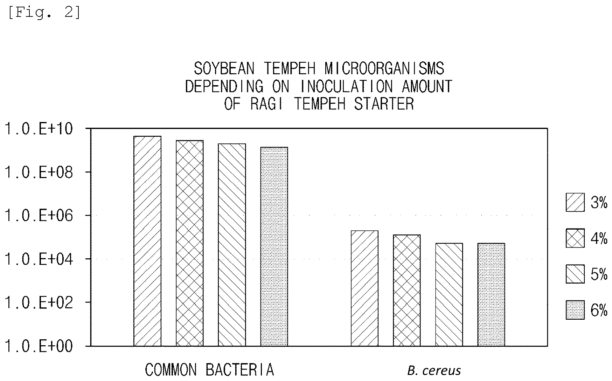

Specifically, the total bacterial count was obtained by smearing a specimen on a standard agar medium and counting the viable bacterial count, and *Bacillus cereus* was counted after smearing a specimen on a selected medium. As a result, as shown in the following Table 1 and FIG. 2, as the inoculation amount of a tempeh fungus was increased, the growth of a tempeh fungus predominated, so that *Bacillus cereus* and the total bacterial count were decreased.

**[Table 1]**

| Added amount (%) of tempeh starter as compared with weight of raw material soybeans | *B. cereus* (CFU/g) | Total bacterial count (CFU/g) |
|---|---|---|
| 3 wt% | 2.2.E+05 | 6.1.E+09 |
| 4 wt% | 1. 6. E+05 | 4.0.E+09 |
| 5 wt% | 6.0.E+04 | 2.5.E+09 |
| 6 wt% | 5.5.E+04 | 1.9.E+09 |

### 1-2. Evaluation of quality of soybean tempeh depending on added amount of acid

Tempeh was produced in the same manner as in Example 1, except that 4 wt% of a tempeh fungus with respect to the total weight of the raw grain soybeans was added as a starter, and 2 wt% or 4 wt% of vinegar was added to produce tempeh. The inhibition of the growth of microorganisms depending on an acid content was compared by measuring the number of *Bacillus cereus,* which is food poisoning bacteria, the total bacterial count (CFU/g) of microorganisms and the fungal count in a TSA medium including kanamycin in the produced tempeh.

As a result, as shown in the following Table 2, it was confirmed that as the added amount of vinegar was increased in a range of 2 to 4 wt%, the growth of a tempeh fungus predominated, so that the growth of food poisoning bacteria, the microbial total bacterial count, and fungi was decreased to lower a microbial level.

**[Table 2]**

| Experimental group (wt%) | *B. cereus* (CFU/g) | Total bacterial count (CFU/g) | Fungi (yeast, mold) (CFU/g) |
|---|---|---|---|
| Conventional starter 4, vinegar 4 | 1.00.E+ 01 - 02 | 1.00.E+ 06 | 6.50.E+ 04 |
| starter 4 vinegar 2 | 1.00.E+ 04 | 6.50.E+ 07 | 5.00.E+ 05 |

### 1-3. Evaluation of quality of soybean tempeh depending on the type of acids

Soybean tempeh was produced in the same manner as in Example 1, except that the type of acids (white vinegar, citric acid, or lactic acid) was changed with respect to the total weight of the raw grain soybeans. The three types of acids were added in amounts such that the same acidity (10.5±1%) was maintained. In the produced tempeh, the numbers of *Bacillus cereus* which is food poisoning bacteria and common bacteria, the total bacterial count (CFU/g), and the fungal count in a TSA medium including kanamycin were measured, and then microbial levels were compared.

As a results, as shown in the following Table 3, any significant difference in food poisoning bacteria, the total bacterial count of microorganisms, and the fungal count was not shown, and thus, it was confirmed that when an acid having an acidity of about 10% which is appropriate for the growth of a tempeh fungus was added, regardless of the type of added acids, a tempeh fungus grew well to maintain the microbial level low.

**[Table 3]**

| Experimental group (based on 4wt%) | *B. cereus* (CFU/g) | Common bacteria (CFU/g) | Fungi (yeast, mold confirmed) (CFU/g) | Total bacterial count (CFU/g) |
|---|---|---|---|---|
| White vinegar (control) | 1.00.E+04 | 5.80.E+07 | 7.50.E+04 | 6.00.E+07 |
| Citric acid | 6.00.E+04 | 4.55.E+07 | 8.00.E+04 | 3.50.E+07 |
| Lactic acid | 5.50.E+04 | 2.35.E+07 | 1.45.E+05 | 4.15.E+07 |

### 1-4. Evaluation of production efficiency and quality of tempeh depending on tempeh height

Soybean tempeh was produced in the same manner as in Example 1, except that the height of tempeh was changed during tempeh molding.

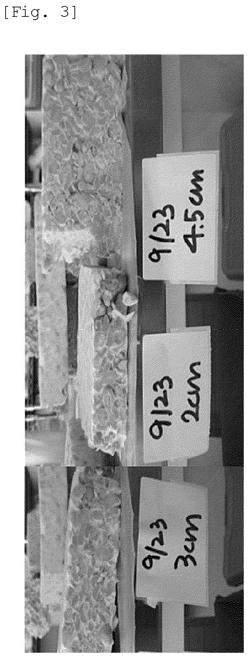

As a result, as shown in the following Table 4 and FIG. 3, when the height of tempeh was less than 4.5 cm, the mycelium of a tempeh fungus was filled even between soybeans in a deep part, the tempeh was hard, and a microbial level such as food poisoning bacteria or common bacteria was maintained low, thereby completing tempeh having good quality. However, when the height of tempeh was more than 4.5 cm, the microbial level such as food poisoning bacteria and the common bacterial count was increased, the mycelium of a tempeh fungus was not spread into the deep part of tempeh, and tempeh which was not hard was completed.

**[Table 4]**

| Height | *B. cereus* (CFU/g) | Common bacteria (CFU/g) |
|---|---|---|
| 2 cm | 1.40.E+04 | 1.15.E+07 |
| 3 cm | 1.60.E+04 | 3.95.E+07 |
| 4.5 cm | 1.40.E+05 | 2.55.E+09 |

### 1-5. Evaluation of quality of soybean tempeh depending on tempeh fermentation humidity

Soybean tempeh was produced in the same manner as in Example 1, except that the fermentation humidity during tempeh fermentation was changed to 60%, 70%, or 80%. The total bacterial count of common bacteria of the produced soybean tempeh was counted and the microbial levels were compared.

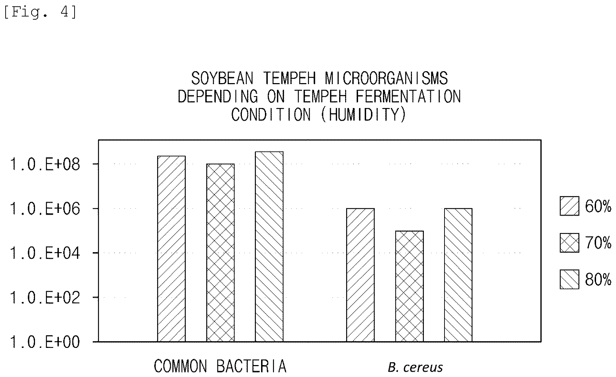

As a result, as shown in the following Table 5 and FIG. 4, the tempeh fungus grew well in all conditions of relative humidity of 60 to 80% to complete tempeh which was hard and had good quality, but the level of food poisoning bacteria and common bacteria was the lowest at an optimal fermentation humidity of a tempeh fungus of 70%.

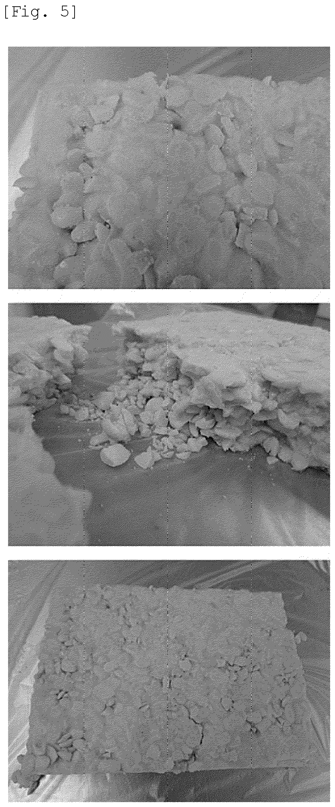

However, in the soybean tempeh produced in the conditions of a relative humidity of 30%, as shown in FIG. 5, a tempeh fungus did not grow well, and thus, white hyphae of a tempeh fungus was not spread to a deep part even visually and fermentation by a tempeh fungus was not performed well. Therefore, tempeh which was not hard and did not maintain the shape, for example, was broken was completed.

From the above results, when fermentation was performed at a relative humidity of 60 to 80%, it was confirmed that good-quality tempeh having hard texture and a low microbial level may be produced efficiently.

**[Table 5]**

| Classification | Common bacteria (CFU/g) | *B. cereus* (CFU/g) |
|---|---|---|
| 60% | 2.2.E+08 | 1.1.E+06 |
| 70% | 1.1.E+08 | 1.1.E+05 |
| 80% | 3.8.E+08 | 1.1.E+06 |

### 1-6. Evaluation of quality of soybean tempeh depending on tempeh fermentation temperature

Soybean tempeh was produced in the same manner as in Example 1, except that the fermentation temperature during tempeh fermentation was changed to 27°C, 30°C, and 33°C. The common bacterial count of the produced soybean tempeh was counted and the microbial level was compared.

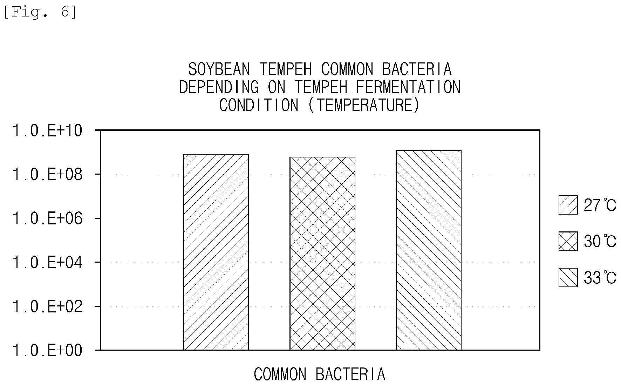

As a result, as shown in the following Table 6 and FIG. 6, a tempeh fungus grew well in all temperature conditions of 27°C to 33°C, but the growth of a tempeh fungus was most dominant at 30°C which is an optimal growth temperature of a tempeh fungus and the common bacterial count was measured low.

**[Table 6]**

| Classification | Common bacteria (CFU/g) |
|---|---|
| 27°C | 8.5.E+08 |
| 30°C | 6.0.E+08 |
| 33°C | 1.2.E+09 |

### 1-7. Evaluation of quality of soybean tempeh depending on steaming method of soybeans

It was evaluated whether the growth of a tempeh fungus and the microbial level of tempeh were changed depending on the steaming method of soybeans.

First, tempeh was produced using soybeans steamed by a steam pressurization method as in Example 1. In addition, soybeans which were soaked in water were boiled in boiling water for 1 hour or more, and then soybeans peeled by hand were used to produce conventional tempeh. The total bacterial count was obtained by counting the viable bacterial count after smearing a specimen on a standard agar medium (MYP medium and TSA medium), and the microbial level of tempeh depending on the steaming method of soybeans was evaluated.

As a result, as shown in the following Table 7, when a rotation steam pressurization type steaming method which is the steaming method of the present disclosure was used, the microbial level was lowered as compared with the case of producing tempeh by a conventional method, and a tempeh fungus grew well, and thus, tempeh having excellent quality was produced.

**[Table 7]**

| Steaming method | MYP medium (CFU/g) | TSA medium (CFU/g) |
|---|---|---|
| Common conventional (boiling method) | 2.E+06 | 1.E+09 |
| Example 1 (Steam pressurization method) | 8.0.E+05 | 3.E+07 |

### 1-8. Evaluation of quality of tempeh depending on type of beans

Common bacteria and food poisoning bacteria in soybean tempeh produced in Example 1 and black bean tempeh produced in Example 3 were counted and microbial levels were compared.

As a result, as shown in the following Table 8, it was confirmed that the common bacterial count was detected as 10⁹ CFU/g or less and the number of *Bacillus cereus* was detected as 10⁴ CFU/g or less, and thus, the microbial level was lowered in all of the bean tempeh produced by the production method of the present disclosure. Thus, it was confirmed that in the tempeh produced by the production method of the present disclosure, a tempeh fungus grew well, had hard and good quality, and had excellent microbial stability, regardless of the type of beans.

**[Table 8]**

| | Common bacteria (CFU/g) | *B. cereus* (CFU/g) |
|---|---|---|
| Soybean | 3.5.E+08 | 1.4.E+03 |
| Black bean | 1.0.E+06 | 1.2.E+03 |

### [Experimental Example 2] Evaluation of aged soybean tempeh

The aging degree and pH of the aged soybean tempeh produced according to Example 2 were measured.

### 2-1. Evaluation of aging degree of aged soybean tempeh depending on inoculation amount of tempeh fungus

It was confirmed whether the aging degree of the aged product was changed when the soybean tempeh was aged, depending on the inoculation amount of a tempeh fungus during soybean tempeh production.

First, soybean tempeh was produced according to the method described in Example 1, except that the added amount of tempeh starter inoculated into the steamed soybeans was changed into 4 wt%, 5 wt%, and 6 wt%.

Brine having a salinity of 7% was added to the soybean tempeh produced above to produce soybean tempeh having a moisture content of 60%. The soybean tempeh prepared above to which brine was added was stirred and aged for 13 days to produce an aged product, and a formol titration method was used to compare the aging degree and pH of the aged product by aging days depending on the inoculation amount of a tempeh fungus (added amount of tempeh starter).

Specifically, the aging degree was evaluated by measuring the amino-type nitrogen content by the following method. First, the aged soybean tempeh was homogenized, a 1% phenolphthalein solution was added to 50 ml of a sample solution diluted 50 times in distilled water, and titration was performed with a 0.1 N sodium hydroxide solution until the color changed to a light red color. 30 ml of a 17.5% formalin solution was titrated by the same method. The aged soybean tempeh solution and the formalin solution were mixed, and then the amino-type nitrogen content (mg%) was measured with the amount (ml) titrated until a light red color was shown with the 0.1 N sodium hydroxide solution. The numerical value of the measured amino-type nitrogen content (mg%) was indicated as an aging degree and evaluated.

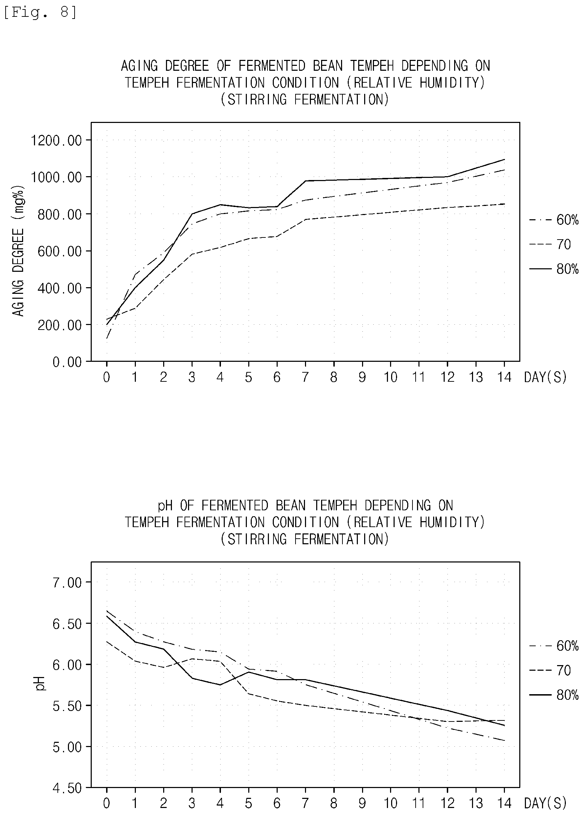

As a result, as shown in the following Table 9 and FIG. 8, until the 5th day of aging, as the inoculation amount of a tempeh fungus was increased, the aging degree was also increased, and as the inoculation amount of a tempeh fungus was increased, the pH of the aged product was decreased. From the results as such, it was confirmed that the aging degree of the aged tempeh may be adjusted depending on the aging period and the inoculation amount of a tempeh fungus (added amount of tempeh starter).

**[Table 9]**

| Aged bean tempeh by added amount of tempeh starter | 4 wt% | | 5 wt% | | 6 wt% | |
|---|---|---|---|---|---|---|
| | pH | Aging degree | pH | Aging degree | pH | Aging degree |
| Day 0 | 6.05 | 100.80 | 6.13 | 91.28 | 6.08 | 81.76 |
| Day 1 | 5.86 | 177.52 | 5.93 | 205.52 | 5.95 | 222.60 |
| Day 2 | 5.77 | 260.96 | 5.86 | 290.92 | 5.89 | 294.56 |
| Day 3 | 5.72 | 290.92 | 5.74 | 390.60 | 5.34 | 381.08 |
| Day 4 | 5.53 | 353.64 | 5.59 | 370.44 | 5.55 | 398.72 |
| Day 5 | 5.59 | 390.60 | 5.36 | 436.52 | 5.41 | 468.16 |
| Day 6 | 5.45 | 510.16 | 5.21 | 468.16 | 5.23 | 490.00 |
| Day 9 | 5.34 | 609.00 | 5.18 | 527.24 | 5.08 | 528.92 |
| Day 13 | 5.50 | 542.08 | 5.21 | 579.74 | 5.05 | 691.88 |

### 2-2. Evaluation of aging degree of aged soybean tempeh depending on fermentation humidity

When soybean tempeh produced with different fermentation humidities during soybean tempeh production was aged, it was confirmed whether the aging degree of the aged product was changed depending on the fermentation humidity.

Soybean tempeh was produced with different fermentation humidities of 60%, 70%, or 80% during tempeh production, brine having a salinity of 7% was added to the soybean tempeh produced to adjust the moisture content of the mixture to 60%, and the aging degree and pH of the aged product which was stirred and aged for 14 days were measured.

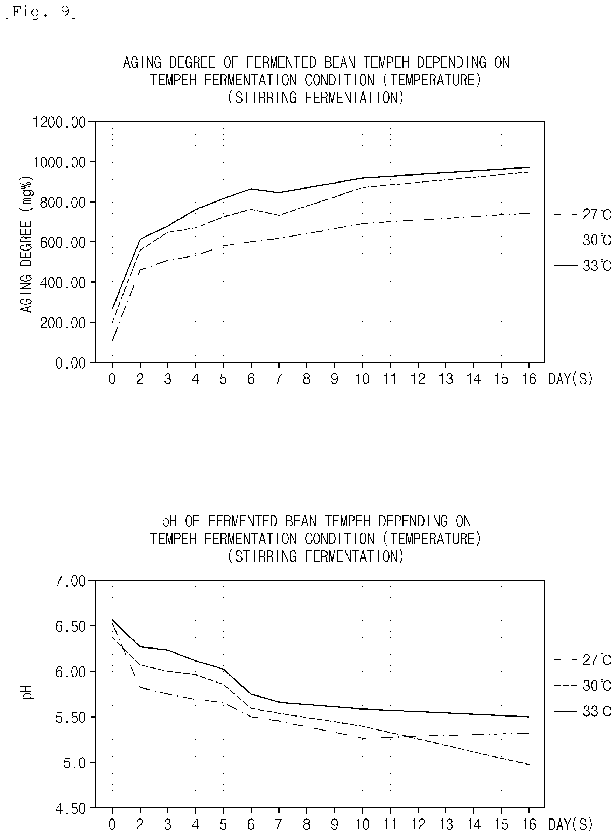

As a result, as shown in the following Table 10 and FIG. 9, a satisfactory aging degree was shown at a humidity of 60% to 80%, and under conditions of humidity lower than or higher than an optimal fermentation humidity of 70%, it was confirmed that bacterial growth was active to show a tendency of increased aging degree. From the results, it was confirmed that the aging degree of the aged tempeh may be adjusted depending on the aging period and the fermentation humidity during tempeh production.

**[Table 10]**

| Aged soybean tempeh by tempeh fermentation conditions (humidity) | Humidity: 60% (conventional) | | Humidity: 70% | | Humidity: 80% | |
|---|---|---|---|---|---|---|
| | pH | Aging degree | pH | Aging degree | pH | Aging degree |
| Day 0 | 6.65 | 126.28 | 6.30 | 216.72 | 6.60 | 210.28 |
| Day 1 | 6.41 | 472.36 | 6.06 | 293.16 | 6.30 | 400.40 |
| Day 2 | 6.30 | 589.68 | 5.97 | 443.24 | 6.20 | 555.24 |
| Day 3 | 6.20 | 748.72 | 6.08 | 586.04 | 5.88 | 802.76 |
| Day 4 | 6.17 | 806.40 | 6.05 | 615.16 | 5.78 | 843.08 |
| Day 5 | 5.95 | 818.16 | 5.66 | 664.72 | 5.91 | 831.88 |
| Day 6 | 5.92 | 824.32 | 5.57 | 679.28 | 5.84 | 836.92 |
| Day 7 | 5.78 | 876.96 | 5.51 | 768.88 | 5.81 | 973.28 |
| Day 12 | 5.24 | 971.88 | 5.32 | 836.92 | 5.44 | 994.56 |
| Day 14 | 5.09 | 1038.24 | 5.32 | 854.84 | 5.27 | 1100.68 |

### 2-3. Evaluation of aging degree of aged soybean tempeh depending on fermentation temperature

When soybean tempeh produced with different fermentation temperature during soybean tempeh production was aged, it was confirmed whether the aging degree of the aged product was changed depending on the fermentation humidity.

Specifically, soybean tempeh was produced with different temperatures of 27°C, 30°C, or 33°C during tempeh fermentation, brine having a salinity of 7% was added to the soybean tempeh produced to adjust the moisture content of the mixture to 60%, and the aging degree and pH of the aged product which was stirred and aged for 14 days were measured.

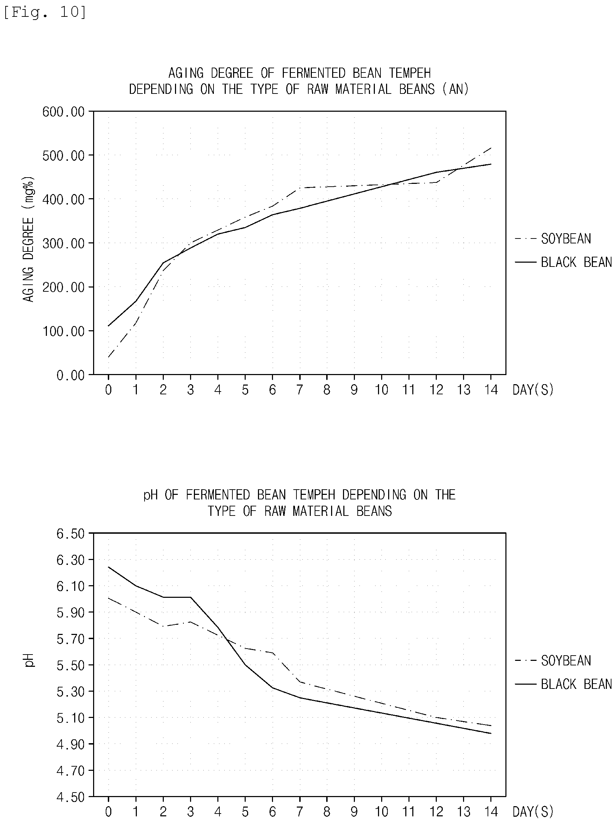

As a result, as shown in the following Table 11 and FIG. 10, it was confirmed that a tempeh fungus grew well in all temperature conditions of 27°C to 33°C, but an aging degree was increased due to bacterial growth at a temperature higher than the optimal growth temperature of a tempeh fungus of 30°C and a tempeh fungus grew less at a temperature lower than 30°C to decrease the aging degree. From the results, it was confirmed that the aging degree of the aged tempeh may be adjusted depending on the aging period and the fermentation temperature during tempeh production.

**[Table 11]**

| Aged soybean tempeh by tempeh fermentation conditions (temperature) | 30°C (conventional) | | 27°C | | 33°C | |
|---|---|---|---|---|---|---|
| | pH | Aging degree | pH | Aging degree | pH | Aging degree |
| Day 0 | 6.40 | 192.08 | 6.56 | 110.04 | 6.53 | 254.80 |
| Day 2 | 6.09 | 560.28 | 5.83 | 452.76 | 6.27 | 610.68 |
| Day 3 | 6.02 | 644.56 | 5.75 | 509.60 | 6.24 | 672.00 |
| Day 4 | 5.98 | 675.64 | 5.69 | 533.96 | 6.12 | 755.72 |
| Day 5 | 5.86 | 725.76 | 5.66 | 585.76 | 6.02 | 812.28 |
| Day 6 | 5.61 | 762.44 | 5.49 | 609.56 | 5.75 | 862.68 |
| Day 7 | 5.53 | 736.12 | 5.46 | 625.24 | 5.67 | 855.68 |
| Day 10 | 5.41 | 873.60 | 5.27 | 694.40 | 5.58 | 918.12 |
| Day 16 | 4.98 | 955.92 | 5.31 | 744.8 | 5.52 | 973 |

### 2-4. Evaluation of aging degree of aged tempeh depending on the type of beans

In order to confirm whether the aging degree of aged tempeh was changed depending on the type of raw material beans, brine having a salinity of 7% was added to each of the soybean tempeh produced in Example 1 and black bean tempeh produced in Example 3 so that the moisture content was about 60%, and aging was performed while stirring was performed at a speed of 50 to 300 rpm at room temperature, and the aging degrees of the produced aged products were compared.

As a result, as shown in the following Table 12 and FIG. 11, the aging degree of aged black bean tempeh was somewhat higher than the aging degree of aged soybean tempeh, but it was confirmed that the pattern of change in aging degree and pH was similar, and thus, aged tempeh may be produced using various types of beans.

**[Table 12]**

| Aged tempeh depending on the type of beans | Soybean | | Black bean | |
|---|---|---|---|---|
| | pH | Aging degree | pH | Aging degree |
| Day 0 | 6.01 | 43.96 | 6.26 | 112.00 |
| Day 1 | 5.91 | 117.32 | 6.12 | 168.00 |
| Day 2 | 5.80 | 235.76 | 6.04 | 252.00 |
| Day 3 | 5.83 | 300.72 | 6.03 | 287.00 |
| Day 4 | 5.74 | 324.80 | 5.80 | 322.00 |
| Day 5 | 5.63 | 354.48 | 5.53 | 336.00 |
| Day 6 | 5.60 | 386.96 | 5.33 | 364.00 |
| Day 7 | 5.38 | 420.56 | 5.27 | 378.00 |
| Day 12 | 5.10 | 439.88 | 5.06 | 459.20 |
| Day 14 | 5.04 | 515.2 | 4.98 | 481.6 |

### [Production Example 1] Production of soybean tempeh-containing sauce

3 wt% to 50 wt% of soybean tempeh produced in Example 1 was added depending on the taste direction of the sauce to be desired, chili pepper powder, chili pepper, salt, soy sauce, tomato ketchup, and also, a composite seasoning food (for example, chili pepper seasoning), sugars (sugar, high fructose, sugar syrup, oligosaccharide, starch syrup), flavoring enhancer, and the like were mixed, sterilized at 70°C to 110°C for 10 minutes to 1 hour, cooled, and packaged to produce a soybean tempeh-containing sauce.

## Claims

1. A method of producing tempeh, the method comprising:
(a) peeling beans;
(b) steaming the peeled beans;
(c) inoculating a tempeh fungus into the steamed beans; and
(d) fermenting the steamed beans inoculated with the tempeh fungus.

2. The method of producing tempeh of claim 1, wherein the peeling in (a) is dry peeling.

3. The method of producing tempeh of claim 1 or claim 2, wherein the steaming of beans in (b) is performed by steam pressurization.

4. The method of producing tempeh of claim 3, wherein the steam is saturated steam.

5. The method of producing tempeh of claim 4, wherein a pressure of the saturated steam pressurization is 0.5 kgf/cm² to 3 kgf/cm².

6. The method of producing tempeh of any one of claims 1 to 5, further comprising adding an acid to the steamed beans.

7. The method of producing tempeh of any one of claims 1 to 6, wherein (c) is adding a tempeh starter in an amount of 2 wt% to 8 wt% with respect to a total weight of the steamed beans.

8. The method of producing tempeh of any one of claims 1 to 7, further comprising molding the beans inoculated with the tempeh fungus into a shape having a height of 2 cm or more and 4.5 cm or less, in (d).

9. The method of producing tempeh of any one of claims 1 to 8, wherein the fermenting in (d) is performed at a temperature of 25°C to 35°C.

10. The method of producing tempeh of any one of claims 1 to 9, wherein the fermenting in (d) is performed at a humidity of 60% to 80%.

11. The method of producing tempeh of any one of claims 1 to 10, wherein the bean is one or more selected from the group consisting of soybeans, black beans, white beans, chick peas, peas, black soybeans, kidney beans, green beans, green pod kidney beans, yellow kidney beans, red flower kidney beans, lentils, and pintos.

12. Tempeh produced by the method of producing tempeh of any one of claims 1 to 11.

13. A sauce comprising the tempeh of claim 12.

14. A method of producing aged tempeh, the method comprising:
(a) producing tempeh by the method of producing tempeh of any one of claims 1 to 11; and
(b) aging a mixture in which at least one or more of saline and brine are added to the produced tempeh.

15. The method of producing aged tempeh of claim 14, wherein the aging comprises stirring the mixture.

16. Aged tempeh produced by the method of claim 14.

17. A sauce comprising the aged tempeh of claim 16.
